# EUROPEAN PATENT SPECIFICATION

(11) **EP 0 713 397 B1**
(45) Date of publication and mention of the grant of the patent: **11.12.2002**
(21) Application number: 93908381.2
(22) Date of filing: 23.03.1993
(51) Int. Cl.: A61K 39/12, A61K 39/15, A61K 39/155, A61K 39/17, A61K 39/215, A61K 39/255, C12N 7/02

(54) **VACCINE CONTAINING LIVE VIRUS**
IMPFSTOFF ENTHALTEND LEBENDES VIRUS
VACCIN CONTENANT UN VIRUS VIVANT

(30) Priority: 24.03.1992 HU 966992; 17.08.1992 HU 2654792
(43) Date of publication of application: 29.05.1996
(73) Proprietor: United Cancer Research Institute, Alexandria, VA 22307 (US)
(72) Inventor: Csatary, Laszlo K., Fort Lauderdale, FL 33316 (US)
(74) Representative: Wilhelms, Rolf E., Dr.
(86) International application number: US9302441
(87) International publication number: WO93018790

(56) References cited:
- EP-A- 0 138 667
- WO-A-88/00980
- WO-A-91/18089
- US-A- 3 874 999
- US-A- 4 158 054
- US-A- 4 235 876
- US-A- 4 457 916
- US-A- 4 824 668
- US-A- 5 124 148
- DATABASE MEDLINE FILE SERVER STN KARLSRUHE ABSTRACT NO.79079695, UEBA 'RESPIRATORY SYNCYTIAL VIRUS.I.CONCENTRATION ADN PURIFIACTION OF THE INFECTIOUS VIRUS'
- DATABASE MEDLINE FILE SERVER STN KARLSRUHE ABSTRACT NO.84027734, WILLIAMS 'THE SURFACE ACTIVITY OF PVP AND OTHER POLYMERS AND THEIR ANTIHEMOLYTIC CAPACITY'
- DATABASE WPI Section Ch, Week 9039 Derwent Publications Ltd., London, GB; Class B04, AN 90-292811 & HU-A-52 812 (PHYLAXIA OLTOANYAGT)
- DATABASE WPI Section Ch, Week 8435 Derwent Publications Ltd., London, GB; Class B04, AN 84-215368 & HU-A-32 250 (PHYLAXIA OLTOANYAG)
- DATABASE WPI Section Ch, Week 8609 Derwent Publications Ltd., London, GB; Class B04, AN 86-055952 & DD-A-229 031 (FORSCH LUNGEN TUBER)
- H. FRAENKEL-CONRAT et al., "Virology", published 1982 by PRENTICE-HALL INC (N.J.), pages 17-20, see entire document.

## Description

The present invention relates to a purified virus vaccine and the purification procedure therefor for the therapy of viral diseases and malignancies.

Hungarian Patents #197 517 and #197 846 describe the use of certain live, apathogenic viruses in the therapy of various human diseases of viral origin. Thus, patent # 197 517 provides a pharmaceutical product containing attenuated Newcastle disease virus suitable for the therapy of herpes, rabies, AIDS and malignancies. Patent # 197 846 describes a pharmaceutical product containing attenuated Gumboro virus suitable for the treatment of hepatitis, rabies, and other diseases of viral origin and malignancies. Although both Gumboro and Newcastle disease viruses cause poultry diseases, the vaccines containing these attenuated viruses are in commercial use. The above patents describe the therapeutic application of these vaccines.

Cryobiology, vol. 20 p.521-526, (1983) discloses that hydroxyethyl starch is a cryoprotective agent.

Since the purity of veterinary vaccines do not meet human purity requirements, infections and complications may result as untoward side effects. Moreover, the stability of veterinary vaccines may also be poor. The present invention is intended to provide a process to obtain purified, lyophilized virus-containing products which are stable for long periods without apparent loss of effectiveness.

Recently, it has been found that other apathogenic viruses can also be used in the therapy of human diseases of viral origin. Any attenuated virus apathogenic for humans can be used, alone or in combination, in the treatment of viral diseases. These may be veterinary, in particular, fowl viruses, or human viruses; e.g.; avian paramyxovirus, avian herpesvirus, avian rotavirus, avian bronchitis, avian encephalitis, avian bursitis (Gumboro) virus, Marek's disease virus, parvovirus, Newcastle disease virus as well as human paramyxovirus, human parvovirus and human adenovirus.

The invention relates to vaccines containing attenuated viruses apathogenic to humans which are effective in the treatment of diseases of viral origin and malignancies, e.g., as follows: AIDS, carcinoma of the rectum, bladder, breast, colon, cervix, esophagus, pancreas, bronchus, liver, kidney and stomach, gynecological cancers, head and neck cancers, lymphomas, malignant melanoma, myeloma, immune deficiency due to irradiation, multiple sclerosis, influenza, common cold and related diseases of viral origin, herpes genitalis and labialis, warts, collagen diseases, acute and chronic hepatitis (B and C), and symptoms following bone marrow transplantation.

The viruses suitable for the above therapeutic purposes may be obtained as usual, e.g., from fibroblast or other cell line cultures or allanto-amniotic fluid of egg embryos. The allanto-amniotic fluid can be obtained from infected hen eggs. The fluid is purified by centrifugation and the supernatant is pelleted by ultracentrifuge. The sediment is rehydrated and sedimented over sucrose gradient, ultracentrifuged again and the pellet is rehydrated and lyophilized.

In a preferred embodiment of the invention the allantoic fluid is centrifuged by approximately 5000 x g, the pellet is discarded and the supernatant is used (if necessary a filtration step can be included). The virus is pelleted from this supernatant by ultracentrifugation (the ultracentrifugation depends on the r.p.m. and time, and may vary over a wide range, usually 35 000 x g for 1 hour). The supernatant is discarded and the pellet is resuspended in a small volume of buffer solution. For appropriate homogeneity a relative longer period of mixing is required.

This homogeneous suspension is layered over a high concentration of sucrose and ultracentrifuged at 90 000 - 100 000 x g (minimal g: 60 000). The supernatant is discarded and the pellet is rehydrated and lyophilized.

In the invention the virus preparation is further immobilised. Protective colloids, either alone or in combination, during lyophilization are generally used in the prior art production of vaccines. Such colloids are, e.g., milk (3-10%), polyvinylpyrrolidone and gelatin (0.1-0.2%), and glucose, sucrose or dextran (1-10%). However, for human use, these colloids are either unsatisfactory or may cause side effects.

We have found that modified hydroxyethyl starch, either alone or in combination, can preferably be used as the protective colloid, (molecular weight: 100 000 - 300 000). Hydroxyethyl-starch of an average molecular weight of 200 000 is used as plasma expander, but such compounds have hitherto not been used as protective colloids for vaccine production.

The new stabilized product according to this invention contains, together with other compounds, an effective amount of hydroxyethyl starch as the protective colloid.

The invention will be detailed in the following examples. Newcastle disease and Gumboro virus can be purchased from Phylaxia of Budapest, Hungary as PHYLAVAC and GUMBOPHYL, respectively.

### Example #1. Purification of Newcastle disease virus from allantois fluid

Three liters of allanto-amniotic fluid containing the virus were centrifuged at 5000 x g for 1 hour. The supernatant was filtered through multiple layers of gauze. The virus was pelleted from the supernatant by ultracentrifugation (SCP 85 H2 ultracentrifuge, RP 19 rotor, 18 500 rpm (35 000 x g, 4°C, 1h)). After discarding the supernatant, the pellet was resuspended in 30 ml NTE buffer (0.15 M NaCl, 0.001 M EDTA, 0.05 M TRIS; pH 7.4). The suspension was gently mixed for 24 hours in an ice bath.

The suspension was further purified by sucrose gradient ultracentrifugation. Thirty ml of 30% (w/w) (=33 % w/v) sucrose in NTE buffer was placed into centrifuge tubes and 5 ml of suspension was layered onto the sucrose. The tubes were ultracentrifuged in an SRP rotor at 95 000 x g (27 500 rpm) for 80 min.

After discarding the supernatants, the pellets were resuspended in NTE buffer (0.5 ml/tube). The collected supernatants were gently mixed for 24 hours in an ice bath.

The concentration of virus during the purification procedure was checked by neuraminidase activity, hemagglutination and ELISA. The infectivity of the virus was measured by the inoculation of preincubated eggs. The protein concentration was measured by the method of Spector. The purity of the product was checked by SDS gel electrophoresis; except for HN, NP and M proteins no other bands (contaminants) should be seen.

The above method displayed the following features:

| | Volume | ELISA (HI) | yield % |
|---|---|---|---|
| Original material | 3 l | 154 | 100 |
| Supernatant | 3 l | 1 | 0.06 |
| Resuspended pellet | 42 ml | 9531 | 87 |
| Supernatant over sucrose | 310 ml | 467 | 31 |
| Purified virus | 11 ml | 20803 | 50 |

### Example #2. Purification of Gumboro virus from Vero cell culture

2300 ml supernatant of Vero cell culture was centrifuged for 30 min at 5000 x g at 4°C. Virus was pelleted from the supernatant by ultracentrifugation (SCP 85 H2 ultracentrifuge, RP 19 rotor, 18 500 rpm (35 000 x g, 4°C, 1h)). After discarding the supernatant, the pellets were resuspended in 23 ml NTE buffer (1% of the original volume). The suspension was gently mixed for 24 hours in an ice bath.

The suspension was further purified by sucrose gradient ultracentrifugation. Thirty ml of 30% (w/w) (=33% w/v) sucrose in NTE buffer was placed into centrifuge tubes and 5 ml of suspension was layered onto the sucrose. The tubes were ultracentrifuged in SRP 28SA rotor at 95 000 x g (27 500 rpm) for 80 min.

After discarding the supernatants, the pellets were resuspended in NTE buffer (1 ml/tube), then washed with 1 ml buffer. The collected supernatants were gently mixed for 24 hours in an ice bath.

The concentration of virus during the purification procedure was checked by ELISA. The infectivity of the virus was measured by its cytopathogenic effect. The protein concentration was measured by the method of Spector.

The above described method displays the following features:

| | volume | ELISA (HI) | yield % |
|---|---|---|---|
| Original material | 2300 ml | 171 | 100 |
| After centrifugation | 2300 ml | 133 | 78 |
| Supernatant | 2300 ml | 48 | 28 |
| Resuspended pellet | 28 ml | 3621 | 26 |
| Supernatant over sucrose | 180 ml | 212 | 10 |
| Purified virus | 13 ml | 5271 | 17 |

### Example #3. Stabilized virus for human therapeutic use

2-2% (v/v) glucose, sucrose and hydroxyethyl-starch (mw: 200 000) (ISOHES, HES 200/0.5) were added to the virus suspension obtained from example #1, then lyophilized. After reconstitution, even after prolonged storage, the original ELISA titre was obtained.

## Claims

1. A method for producing a highly pure, stable vaccine, including the steps of:
(a) obtaining a fluid containing the selected virus;
(b) subjecting the virus-containing fluid to centrifugation to produce a supernatant containing the selected virus;
(c) subjecting the supernatant to ultracentrifugation to produce a virus-containing pellet;
(d) forming a homogeneous virus-containing suspension by mixing the pellet in a buffer solution in an ice bath;
(e) purifying said suspension by layering the suspension onto about 30 % (w/w) sucrose and subjecting said sucrose layered suspension to ultracentrifugation at 60,000-100,000 x g to produce a virus-containing pellet;
(f) collecting the virus-containing pellet, resuspending the pellet in a buffer solution and mixing the pellet-buffer solution in an ice bath to produce a virus suspension;
(g) stabilizing the virus suspension by adding a protective colloid to the virus suspension, that protective colloid comprising a modified hydroxyethyl starch having a molecular weight of 100.000 to 300.000; and
(h) lyophilizing the stabilized virus suspension to produce a stable, highly pure vaccine suitable for administration to humans.

2. The method described in claim 1, wherein said virus is selected from the group of viruses apathogenic to humans consisting of avian paramyxovirus, avian herpes virus, avian rotavirus, avian bronchitis virus, avian encephalitis virus, avian bursitis virus, Marek's disease virus, parvovirus, Newcastle disease virus and mixures thereof.

3. The method described in claim 1, wherein the fluid containing the selected virus is obtained from a fluid source selected from the group consisting of fibroblast culture supernatants, cell line culture supernatants and allanto-amniotic fluid.

4. The method described in claim 1, wherein said selected virus is pathogenic to humans and is selected from the group consisting of human paramyxovirus, human parvovirus, human adenovirus and mixtures thereof.

5. Method for producing the vaccine including the following steps:
(a) obtaining a fluid containing the selected virus;
(b) centrifuging the virus-containing fluid to produce a virus-containing supernatant;
(c) subjecting the virus-containing supernatant to ultra centrifugation at about 35,000 x g to produce a virus-containing pellet;
(d) mixing the virus-containing pellet in a buffer solution in an ice bath to form a homogeneous virus-containing suspension;
(e) purifying said virus-containing suspension in a sucrose gradient in an ultracentrifuge ar 60,000-100,000 x g to produce a highly pure virus-containing pellet;
(f) forming a highly pure virus-containing suspension by mixing said virus-containing pellet in a buffer solution in an ice bath;
(g) stabilizing said highly pure, virus-containing suspension by adding hydroxyethyl starch having a molecular weight of 100,000 to 300,000 to said suspension; and
(h) lyophilizing said stabilized, highly pure virus-containing suspension to form a stable, highly pure viral vaccine suitable for administration to humans.

6. The method described in claim 3, wherein said fluid is allanto-amniotic fluid and said selected virus is Newcastle disease virus.

7. The method described in claim 3, wherein said fluid is cell line culture supernatant and said selected virus is avian bursitis (Gumboro) disease virus.

8. The method described in claim 1, wherein steps (d) and (f) are conducted at a pH of about 7.4 in a solution of NTE buffer.

9. A highly purified, stable human vaccine produced according to the method of claim 1.

10. A highly purified, stable vaccine for treating human diseases of viral origin and malignancies produced from a selected virus apathogenic to humans produced by a process comprising the steps of:
(a) obtaining a fluid containing the selected virus;
(b) centrifuging the virus-containing fluid to produce a virus-containing supernatant;
(c) subjecting the virus-containing supernatant to ultra centrifugation at about 35,000 x g to produce a virus-containing pellet;
(d) mixing the virus-containing pellet in a buffer solution in an ice bath to form a homogeneous virus-containing suspension;
(e) purifying said virus-containing suspension in a sucrose gradient in an ultracentrifuge at 60,000-100,000 x g to produce a highly pure virus-containing pellet;
(f) forming a highly pure virus-containing suspension by mixing said virus-containing pellet in a buffer solution in an ice bath;
(g) stabilizing said highly pure, virus-containing suspension by adding hydroxyethyl starch having a molecular weight of 100,000 to 300,000 to said suspension; and
(h) lyophilizing said stabilized, highly pure virus-containing suspension to form a stable, highly pure viral vaccine suitable for administration to humans.

11. The highly purified vaccine of claim 10, wherein said selected virus is selected from the group consisting of avian paramyxovirus, avian herpesvirus, avian rotavirus, avian bronchitis virus, avian encephalitis virus, avian bursitis (Gumboro) virus, Marek's disease virus, parvovirus, Newcastle disease virus and mixtures thereof.

12. The highly purified vaccine of claim 10, wherein the selected virus is pathogenic to humans and is selected from the group consisting of human paramyxovirus, human parvovirus, human adenovirus and mixtures thereof.

13. The highly purified vaccine of claim 11, wherein said selected virus is Newcastle disease virus.

14. The highly purified vaccine of claim 11, wherein said selected virus is avian bursitis (Gumboro) disease virus.

15. The highly purified vaccine of claim 10, wherein steps (d) and (f) are conducted at a pH of about 7.4 in a solution of NTE buffer.

## Patentansprüche

1. Verfahren zur Herstellung eines hochreinen, stabilen Impfstoffs mit den Stufen:
(a) Erhalten eines Fluids, welches das ausgewählte Virus enthält;
(b) Zentrifugieren des virushaltigen Fluids zur Herstellung eines Überstands, welcher das ausgewählte Virus enthält;
(c) Ultrazentrifugieren des Überstands zur Herstellung eines virushaltigen Pellets;
(d) Bilden einer homogenen virushaltigen Suspension durch Mischen des Pellets in einer Pufferlösung in einem Eisbad;
(e) Reinigen der Suspension durch Schichten der Suspension auf ungefähr 30 %ige (w/w) Sucrose und Ultrazentrifugieren der auf Sucrose geschichteten Suspension bei 60.000 - 100.000 x g zur Herstellung eines virushaltigen Pellets;
(f) Sammeln des virushaltigen Pellets, Resuspendieren des Pellets in einer Pufferlösung und Mischen der Pellet-Pufferlösung in einem Eisbad zur Herstellung einer Virussuspension;
(g) Stabilisieren der Virussuspension durch Zugabe eines Schutzkolloids zu der Virussuspension, wobei das Schutzkolloid eine modifizierte Hydroxyethylstärke mit einem Molekulargewicht von 100.000 - 300.000 enthält, und
(h) Lyophilisieren der stabilisierten Virussuspension zur Herstellung eines stabilen, hochreinen Impfstoffs, der zur Verabreichung an Menschen geeignet ist.

2. Verfahren gemäß Anspruch 1, wobei es sich bei dem Virus um eines der für den Menschen nicht pathogenen Viren Vogelparamyxovirus, Vogelherpesvirus, Vogelrotavirus, Vogelbronchitisvirus, Vogelencephalitisvirus, Vogelbursitisvirus, Mareks-Disease-Virus, Parvovirus, Newcastle-Disease-Virus oder eine Mischung davon handelt.

3. Verfahren gemäß Anspruch 1, wobei das Fluid, welches das ausgewählte Virus enthält, aus dem Überstand von Fibroblastenkulturen, aus dem Überstand von Zelllinienkulturen und/oder einem allanto-amniotischen Fluid als Fluidquelle erhalten wird.

4. Verfahren gemäß Anspruch 1, wobei das ausgewählte Virus für den Menschen pathogen ist und es sich dabei um das humane Paramyxovirus, humane Parvovirus, humane Adenovirus oder Mischungen davon handelt.

5. Verfahren zur Herstellung eines Impfstoffs mit den Stufen:
(a) Erhalten eines Fluids, welches das ausgewählte Virus enthält;
(b) Zentrifugieren des virushaltigen Fliuds zur Herstellung eines virushaltigen Überstands;
(c) Ultrazentrifugieren des virushaltigen Überstands bei etwa 35.000 x g zur Herstellung eines virushaltigen Pellets;
(d) Mischen des virushaltigen Pellets in einer Pufferlösung in einem Eisbad zur Bildung einer homogenen virushaltigen Suspension;
(e) Reinigen der virushaltigen Suspension in einem Sucrosegradienten in einer Ultrazentrifuge bei 60.000 - 100.000 x g zur Herstellung eines hochreinen virushaltigen Pellets;
(f) Bilden einer hochreinen virushaltigen Suspension durch Mischen des virushaltigen Pellets in einer Pufferlösung in einem Eisbad;
(g) Stabilisieren der hochreinen virushaltigen Suspension durch Zugabe von Hydroxyethylstärke mit einem Molekulargewicht von 100.000- 300.000 zu der Suspension; und
(h) Lyophilisieren der stabilisierten, hochreinen virushaitigen Suspension zur Bildung eines stabilen, hochreinen viralen Impfstoffs, der zur Verabreichung an Menschen geeignet ist.

6. Verfahren gemäß Anspruch 3, wobei es sich bei dem Fluid um allanto-amniotisches Fluid handelt und das ausgewählte Virus das Newcastle-Disease-Virus ist.

7. Verfahren gemäß Anspruch 3, wobei es sich bei dem Fliud um den Überstand einer Zelllinienkultur handelt und das ausgewählte Virus das Vogelbursitis(Gumboro)-Disease-Virus ist.

8. Verfahren gemäß Anspruch 1, wobei die Stufen (d) und (f) bei einem pH-Wert von etwa 7,4 in einer NTE-Pufferlösung durchgeführt werden.

9. Hochgereinigter, stabiler humaner Impfstoff, welcher nach dem Verfahren gemäß Anspruch 1 hergestellt wurde.

10. Hochgereinigter stabiler Impfstoff zur Behandlung von humanen Erkrankungen viralen Ursprungs und Malignitäten, hergestellt aus einem ausgewählten, für den Menschen nicht pathogenen Virus durch ein Verfahren mit den Stufen:
(a) Erhalten eines Fluids, welches das ausgewählte Virus enthält;
(b) Zentrifugieren des virushaltigen Fluids zur Herstellung eines virushaltigen Überstands;
(c) Ultrazentrifugieren des virushaltigen Überstands bei etwa 35.000 x g zur Herstellung eines virushaltigen Pellets;
(d) Mischen des virushaltigen Pellets in einer Pufferlösung in einem Eisbad zur Bildung einer homogenen virushaltigen Suspension;
(e) Reinigen der virushaltigen Suspension in einem Sucrosegradienten in einer Ultrazentrifuge bei 60.000 - 100.000 x g zur Herstellung eines hochreinen virushaltigen Pellets;
(f) Bilden einer hochreinen virushaltigen Suspension durch Mischen des virushaltigen Pellets in einer Pufferlösung in einem Eisbad;
(g) Stabilisieren der hochreinen virushaltigen Suspension durch Zugabe von Hydroxyethylstärke mit einem Molekulargewicht von 100.000 - 300.000 zu der Suspension; und
(h) Lyophilisieren der stabilisierten, hochreinen virushaltigen Suspension zur Bildung eines stabilen, hochreinen viralen Impfstoffs, der zur Verabreichung an Menschen geeignet ist.

11. Hochgereinigter Impfstoff gemäß Anspruch 10, wobei es sich bei dem Virus um ein Vogelparamyxovirus, Vogelherpesvirus, Vogelrotavirus, Vogelbronchitisvirus, Vogelencephalitisvirus, Vogelbursitis(Gumboro)virus, Mareks-Disease-Virus, Parvovirus, Newcastle-Disease-Virus oder Mischungen davon handelt.

12. Hochgereinigter Impfstoff gemäß Anspruch 10, wobei das ausgewählte Virus für Menschen pathogen ist und es sich dabei um ein humanes Paramyxovirus, humanes Parvovirus, humanes Adenovirus oder Mischungen davon handelt.

13. Hochgereinigter Impfstoff gemäß Anspruch 11, wobei es sich bei dem ausgewählten Virus um das Newcastle-Disease-Virus handelt.

14. Hochgereinigter Impfstoff gemäß Anspruch 11, wobei es sich bei dem Virus um das Vogelbursitis(Gumboro)-Disease-Virus handelt.

15. Hochgereinigter Impfstoff gemäß Anspruch 10, wobei die Stufen (d) und (f) bei einem pH-Wert von etwa 7,4 in einer NTE-Pufferlösung durchgeführt werden.

## Revendications

1. Procédé destiné à produire un vaccin hautement purifié, stable, comprenant les étapes consistant à obtenir :
(a) obtenir un liquide contenant le virus sélectionné ;
(b) soumettre le liquide contenant le virus à une centrifugation pour produire un surnageant contenant le virus sélectionné ;
(c) soumettre le surnageant à une ultracentrifugation de façon à produire une pastille contenant le virus ;
(d) former une suspension homogène contenant le virus en mélangeant la pastille à une solution tampon dans un bain de glace ;
(e) purifier ladite suspension en plaçant la suspension sur une couche de saccharose à environ 30% (p/p) et soumettre ladite suspension placée sur couche de saccharose à une ultracentrifugation à 60 000-100 000 x g pour produire une pastille contenant le virus ;
(f) reprendre la pastille contenant le virus, remettre en suspension la pastille dans une solution tampon et mélanger la solution pastille-tampon dans un bain de glace pour produire une suspension de virus ;
(g) stabiliser la suspension de virus en ajoutant un colloïde protecteur à la suspension de virus, ce colloïde protecteur contenant un amidon hydroxyethylé modifié, d'un poids moléculaire de 100 000 à 300 000 ; et
(h) lyophiliser la suspension de virus stabilisée de façon à produire un vaccin hautement purifié, stable, adapté à une administration à l'homme.

2. Procédé selon la revendication 1, dans lequel ledit virus est sélectionné parmi le groupe des virus non pathogènes pour l'homme consistant en le paramyxovirus aviaire, le virus herpès aviaire, le rotavirus aviaire, le virus de la bronchite aviaire, le virus de l'encéphalomyélite aviaire, le virus de la bursite aviaire, le virus de la maladie de Marek, le parvovirus, du virus de la maladie de Newcastle et les mélanges de ces virus.

3. Procédé selon la revendication 1, dans lequel le liquide contenant le virus sélectionné est obtenu à partir d'une source de liquides choisis parmi le groupe consistant en les surnageants de cultures de fibroblastes, les surnageants de cultures de lignées cellulaires et le liquide allanto-amniotique.

4. Procédé selon la revendication 1, dans lequel ledit virus sélectionné est pathogène pour l'homme et est sélectionné parmi le groupe consistant en le paramyxovirus humain, le parvovirus humain, les adénovirus humains et les mélanges de ces virus.

5. Procédé pour produire un vaccin incluant les étapes suivantes :
(a) obtenir un liquide contenant le virus sélectionné ;
(b) centrifuger le liquide contenant le virus de façon à produire un surnageant contenant le virus ;
(c) soumettre le surnageant contenant le virus à une ultracentrifugation de l'ordre de 35 000 x g pour produire une pastille contenant le virus ;
(d) mélanger la pastille contenant le virus à une solution tampon dans un bain de glace pour former une suspension homogène contenant le virus ;
(e) purifier ladite suspension contenant le virus en gradient de saccharose dans une ultracentrifugeuse à 60 000-100 000 x g de façon à produire une pastille hautement purifiée contenant le virus ;
(f) former une suspension hautement purifiée contenant le virus en mélangeant ladite pastille contenant le virus dans une solution tampon dans un bain de glace ;
(g) stabiliser ladite suspension hautement purifiée contenant le virus en ajoutant à ladite suspension un amidon hydroxyéthyé d'un poids moléculaire de 100 000 à 300 000 ; et
(h) lyophiliser ladite suspension contenant le virus hautement purifiée et stabilisée, pour former un vaccin viral hautement purifié, stable, adapté à une administration à l'homme.

6. Procédé selon la revendication 3, dans lequel ledit liquide est le liquide allanto-amniotique et où ledit virus sélectionné est le virus de la maladie de Newcastle.

7. Procédé selon la revendication 3, dans lequel ledit liquide est le surnageant de culture de lignées cellulaires et où ledit virus sélectionné est le virus de la maladie de la bursite aviaire (Gumboro).

8. Procédé selon la revendication 1, dans lequel les étapes (d) et (f) sont effectuées à un pH d'environ 7,4 dans une solution tampon NTE.

9. Vaccin humain stable hautement purifié produit selon le procédé de la revendication 1.

10. Vaccin stable hautement purifié pour le traitement des maladies humaines d'origine virale et les malignités produites à partir d'un virus sélectionné non pathogène pour l'homme produit par un processus comprenant les étapes consistant à :
(a) obtenir un liquide contenant le virus sélectionné ;
(b) centrifuger le liquide contenant le virus de façon à produire un surnageant contenant le virus ;
(c) soumettre le surnageant contenant le virus à une ultracentrifugation à environ 35 000 x g de façon à produire une pastille contenant le virus ;
(d) mélanger la pastille contenant le virus à une solution tampon dans un bain de glace de façon à former une suspension homogène contenant le virus ;
(e) purifier ladite suspension contenant le virus en gradient de saccharose dans une ultracentrifugeuse à 60 000-100 000 x g de façon à produire une pastille hautement purifiée contenant le virus ;
(f) former une suspension hautement purifiée contenant le virus en mélangeant ladite pastille contenant le virus dans une solution tampon dans un bain de glace ;
(g) stabiliser ladite suspension contenant le virus hautement purifiée en ajoutant à ladite suspension un amidon hydroxyéthylé d'un poids moléculaire de 100 000 à 300 000 ; et
(h) lyophiliser ladite suspension contenant le virus hautement purifiée et stabilisée, de façon à former un vaccin viral hautement purifié, stable, adapté à une administration à l'homme.

11. Vaccin hautement purifié selon la revendication 10, dans lequel ledit virus sélectionné est sélectionné dans le groupe consistant en le paramyxovirus aviaire, le virus herpès aviaire, le rotavirus aviaire, le virus de la bronchite aviaire, le virus de l'encéphalomyélite aviaire, le virus de la bursite aviaire (Gumboro), le virus de la maladie de Marek, le parvovirus, le virus de la maladie de Newcastle et les mélanges de ces virus.

12. Vaccin hautement purifié selon la revendication 10, dans lequel ledit virus sélectionné est pathogène pour l'homme et est sélectionné dans le groupe consistant en le paramyxovirus humain, le parvovirus humain, les adénovirus humain et les mélanges de ces virus.

13. Vaccin hautement purifié selon la revendication 11, dans lequel ledit virus sélectionné est le virus de la maladie de Newcastle.

14. Vaccin hautement purifié selon la revendication 11, dans lequel ledit virus sélectionné est le virus de la maladie de la bursite aviaire (Gumboro).

15. Vaccin hautement purifié selon la revendication 10, dans lequel les étapes (d) et (f) sont effectuées à un pH d'environ 7,4 dans une solution tampon NTE.
